# EUROPÄISCHE PATENTSCHRIFT

(11) **EP 2 710 972 B1**
(45) Veröffentlichungstag und Bekanntmachung des Hinweises auf die Patenterteilung: **13.04.2016**
(21) Anmeldenummer: 13181138.2
(22) Anmeldetag: 21.08.2013
(51) Int. Cl.: A61B 17/88, A61B 17/00, B05C 17/015, F16K 5/04, F16K 11/085, A61M 39/22

(54) **Drehventilkörper für Austragsvorrichtung für Knochenzemente**
Rotating valve body for a discharge device for bone cements
Corps de vanne rotatif pour dispositif d'extraction pour ciments osseux

(30) Priorität: 20.09.2012 DE 102012018596
(43) Veröffentlichungstag der Anmeldung: 26.03.2014
(73) Patentinhaber: Heraeus Medical GmbH, 61273 Wehrheim (DE)
(72) Erfinder: Vogt, Sebastian, 99092 Erfurt (DE); Greiner, Clemens, 30826 Garbsen (DE); Hein, Rudolf, 31535 Neustadt (DE)
(74) Vertreter: Panten, Kirsten

(56) Entgegenhaltungen:
- EP-A1- 1 607 694
- EP-A2- 2 267 345
- DE-A1-102010 019 222
- US-A- 4 328 833
- US-A1- 2006 208 000

## Beschreibung

Die Erfindung betrifft Drehventilkörper für Austragsvorrichtungen für Knochenzemente. Gegenstand der Erfindung ist auch ein preisgünstig herzustellendes Drehventil, das in druckgasbetriebenen medizinischen Geräten zur Regulierung des Druckgasstroms bestimmt ist, wobei die aus Kunststoff gefertigten medizinischen Geräte zum einmaligen Gebrauch vorgesehen sind.

In der Orthopädie und Unfallchirurgie werden PMMA-Knochenzemente sehr häufig zur mechanischen Fixierung von Gelenkendoprothesen verwendet. Bei modernen Vakuummischsystemen wird der Zement in einer Kartusche durch Vermischung des Polymerpulvers mit der Monomerflüssigkeit hergestellt. Der gebildete Zementteig wird anschließend mit Hilfe einer manuell zu betätigenden Auspressvorrichtung aus der Kartusche ausgepresst und appliziert. Der manuelle Auspressvorgang wird von vielen Anwendern wegen der dazu erforderlichen Handkraft als unangenehm empfunden. Deshalb wurden in der DE 10 2010 019 222 A1 sowie der DE 10 2010 019 224 B3 druckgasbetrieben Auspressvorrichtungen vorgeschlagen. Diese Vorrichtungen sind vorzugsweise aus preisgünstigem Kunststoff gefertigt und nur zum einmaligen Gebrauch bestimmt. Bei diesen Vorrichtungen wurde allgemein ein Drehventil vorgeschlagen. Daneben wurden in der nicht vorveröffentlichten DE 10 2012 013 464 Drehventile auch in druckgasbetriebenen Sprühvorrichtungen vorgeschlagen, und in Vorrichtungen zur kurzzeitigen Vakuumerzeugung (Nicht vorveröffentlichte DE 10 2011 117 527) vorgeschlagen, ohne jedoch die Drehventile detailliert zu beschreiben.

Bei der Verwendung von flüssigem Kohlendioxid können Drücke bis 60 bar auftreten, bisher sind zur Regulierung solcher hohen Drücke kostenintensive aus Metall gefertigte Ventile üblich. Diese Ventile sind für eine hohe Lebensdauer und für die Gewährleistung von mehreren Tausenden Öffnungs- und Schließprozessen ausgelegt. Auf Grund der hohen Beschaffungskosten sind aus Metall gefertigte Ventile jedoch für Einmalanwendungen mit nur wenigen Öffnungs- und Schließprozessen nicht sinnvoll und wirtschaftlich möglich.

Die Aufgabe der Erfindung besteht also darin, die Nachteile des Stands der Technik zu überwinden. Insbesondere soll ein kostengünstiges Drehventil bereitgestellt werden, das preisgünstig gefertigt werden kann und wobei der Drehventilkörper bevorzugt nur aus einem einzigen Teil bestehen soll. Das Drehventil soll lagerfähig sein und eine druckgasregulierende Funktion bei einem Druck von 60 bar von maximal 30 Minuten haben. Das Drehventil soll eine Schaltposition haben, in der ein Druckgasstrom blockiert werden kann und eine zweite Schaltposition, in der der Druckgasstrom fließen kann.

Eine weitere Aufgabe der Erfindung besteht darin, ein Drehventil bereit zu stellen, bei dem neben der Regulierung eines Druckgasstroms bei Blockierung des Gasstroms eine synchrone Druckentlastung im Bereich der Rezipientenseite des Drehventils erreicht wird.

Erfindungsgemäß wurde erkannt, dass auf Grund der hohen Beschaffungskosten von aus Metall gefertigten Ventilen, diese für Einmalanwendungen mit nur wenigen Öffnungs- und Schließprozessen nicht sinnvoll sind und wirtschaftlich ungeeignet sind.

Daher wurde die Aufgabe der Erfindung gelöst durch einen Drehventilkörper für eine druckgasbetriebene Austragsvorrichtung für Knochenzemente, der drehbar in einem passenden Ventilsitz in der Austragsvorrichtung anzuordnen ist, wobei der Drehventilkörper in zumindest einem für den Ventilsitz vorgesehenen Bereich um die Drehachse des Drehventilkörpers drehsymmetrisch ist und auf der drehsymmetrischen Mantelfläche dieses Bereichs zumindest eine Dichtnocke aus einem elastischen Kunststoff angeordnet ist, die sich über den Radius der drehsymmetrischen Mantelfläche erhebt, so dass der Umfang des Drehventilkörpers durch die Dichtnocke größer als der Innendurchmesser des Ventilsitzes ist, und wobei in dem Drehventilkörper zumindest eine Durchführung angeordnet ist, die sich von einer ersten Öffnung in der drehsymmetrischen Mantelfläche des Drehventilkörpers im Bereich der Dichtnocke bis in eine zweite Öffnung in der drehsymmetrischen Mantelfläche des Drehventilkörpers erstreckt.

Dabei kann vorgesehen sein, dass auf der drehsymmetrischen Mantelfläche zumindest eine zweite Dichtnocke aus einem elastischen Kunststoff angeordnet ist, die sich über den Radius der drehsymmetrischen Mantelfläche erhebt, so dass der Umfang dies Drehventilkörpers durch die zweite Dichtnocke größer als der Innendurchmesser des Ventilsitzes ist, und wobei in dem Drehventilkörper zumindest eine zweite Durchführung angeordnet ist, wobei sich die zweite Durchführung von einer dritten Öffnung in der drehsymmetrischen Mantelfläche des Drehventilkörpers im Bereich der zweiten Dichtnocke bis in eine vierte Öffnung in der in Bezug auf die Austragsvorrichtung nach außen gerichteten Deckfläche des Drehventilkörpers erstreckt.

Ein solcher Drehventilkörper hat den Vorteil, dass durch die zweite Dichtnocke eine Gasleitung zum Ablassen eines Überdrucks aus der Austragsvorrichtung 'möglich ist. Hierdurch kann der Druck von einem Förderkolben genommen werden, um einen weiteren Austrag eines medizinischen Fluids, wie eines Knochenzements, zu unterbrechen.

Ferner kann vorgesehen sein, dass der gesamte Drehventilkörper zu mehr als 90%, vorzugsweise vollständig aus Kunststoff besteht, vorzugsweise aus dem gleichen Kunststoff aus dem auch die Dichtnocke oder die Dichtnocken bestehen, wobei der Drehventilkörper und die Dichtnocke oder die Dichtnocken besonders bevorzugt einteilig aufgebaut sind.

Hierdurch kann eine besonders kostengünstige Fertigung des Drehventilkörpers erreicht werden.

Gemäß einer Weiterbildung der Erfindung kann auch vorgesehen sein, dass die Dichtnocke oder die Dichtnocken aus Polyoxymethylen, Polyamid oder Teflon besteht oder bestehen und vorzugsweise auch der Drehventilkörper zu mehr als 90%, insbesondere vollständig aus Polyoxymethylen, Polyamid oder Teflon besteht.

Besonders bevorzugt wird Polyoxymethylen als Material für die Dichtnocke und besonders bevorzugt auch des Drehventilkörpers. Das Polyoxymethylen und auch die anderen Materialien haben eine besonders geeignete Elastizität, so dass der Dichtnocke beziehungsweise die Dichtnocken eine Öffnung durch den elastischen Druck auch bei einem Überdruck auf die Dichtnocke noch zuverlässig gasdicht verschließen kann.

Gemäß einer weiteren bevorzugten Ausführungsform der Erfindung kann auch vorgesehen sein, dass die erste Öffnung, bezogen auf die Drehachse des Drehventilkörpers, in der gleichen Höhe wie die erste Dichtnocke angeordnet ist und vorzugsweise die dritte Öffnung, bezogen auf die Drehachse des Drehventilkörpers, in der gleichen Höhe wie die zweite Dichtnocke angeordnet ist.

Hierdurch wird ein einfacher Aufbau des Ventilsitzes ermöglicht, in der Drehventilkörper einzusetzen ist.

Auch kann vorgesehen sein, dass sich die erste Durchführung senkrecht zur Drehachse des Drehventilkörpers durch den Drehventilkörper erstreckt, so dass die erste Öffnung gegenüberliegend zur zweiten Öffnung angeordnet ist.

Durch die große Beabstandung der beiden Öffnungen kann eine hohe Dichtigkeit des eingesetzten Drehventilkörpers erzielt werden.

Ferner kann vorgesehen sein, dass in zumindest einer der Deckflächen des Drehventilkörpers ein axialer Hohlraum angeordnet ist, der nach außen offen ist, wobei die vierte Öffnung in dem Hohlraum angeordnet ist, wobei vorzugsweise der Hohlraum in der in Bezug auf die Austragsvorrichtung nach außen gerichteten Deckfläche des Drehventilkörpers angeordnet ist.

Durch diesen Hohlraum kann Material eingespart werden, so dass der Aufbau des Drehventilkörpers leichter und kostengünstiger wird.

Gemäß einer weiteren bevorzugten Ausführungsform der Erfindung kann auch vorgesehen sein, dass in der drehsymmetrischen Mantelfläche des Drehventilkörpers zumindest zwei tangential umlaufende Nuten vorgesehen sind, wobei vorzugsweise in den Nuten Führungsringe, besonders bevorzugt Kunststoffführungsringe angeordnet sind.

Die Nuten ermöglichen eine Abdichtung des Drehventils, wenn der Drehventilkörper in den Ventilsitz eingesetzt ist. Zudem dienen die Führungsringe zur Führung der Drehung des Drehventilkörpers im Ventilsitz.

Für erfindungsgemäße Drehventilkörpers mit zwei Durchführungen kann bevorzugt auch vorgesehen sein, dass die erste Öffnung und die dritte Öffnung bezogen auf die Drehachse des Drehventilkörpers um mindestens 10° zueinander versetzt und in unterschiedlicher Höhe angeordnet sind, insbesondere zwischen 10° und 90° zueinander versetzt angeordnet sind.

Hierdurch wird eine ausreichende Drehung des Ventilkörpers sichergestellt werden, damit eine einfache und zuverlässige Bedienung des Drehventils möglich ist.

Bei solchen Drehventilkörpern mit zwei Durchführungen kann ferner vorgesehen sein, dass die erste Dichtnocke und die zweite Dichtnocke bezogen auf die Drehachse des Drehventilkörpers um mindestens 10° zueinander versetzt und in unterschiedlicher Höhe angeordnet sind, insbesondere zwischen 15° und 90° zueinander versetzt angeordnet sind, wobei vorzugsweise die erste Dichtnocke bezogen auf die Drehachse in der gleichen Höhe wie die erste Öffnung angeordnet ist, die zweite Dichtnocke bezogen auf die Drehachse in der gleichen Höhe wie die dritte Öffnung angeordnet ist und die Dichtnocken zu den in der gleichen Höhe angeordneten Öffnungen um den gleichen Winkel zueinander versetzt angeordnet sind, wie die Dichtnocken zueinander.

Diese Anordnung der Dichtnocken und Öffnungen zueinander kann die Austragsvorrichtung mit den Gasleitungen hierzu besonders einfach aufgebaut werden, da die Gasleitungen der Austragsvorrichtung nebeneinander in den Ventilsitz münden und eine Entlüftung und die Druckbeaufschlagung durch eine leichte Drehung des Ventilkörpers im Ventilsitz möglich ist.

Bevorzugt kann auch vorgesehen sein, dass in der in Bezug auf die Austragsvorrichtung nach außen gerichteten Deckfläche des Drehventilkörpers wenigstens ein zur Drehachse des Drehventilkörpers nicht symmetrisches Schlüsselelement angeordnet ist, so dass das Drehventilkörper in dem Ventilsitz mit einem Bedienelement, das formschlüssig in das Schlüsselelement greift, drehbar ist.

Durch dieses Schlüsselelement kann eine leichte und sichere Bedienung des mit dem Drehventilkörper aufgebauten Ventils sichergestellt werden.

Dabei kann vorgesehen sein, dass die Schlüsselfläche dreieckig, quadratisch, rechteckig, sechseckig, achteckig oder ellipsenförmig ist, oder aus Kombinationen von Quadraten, Rechtecken, Dreiecken, Ellipsen oder Kreisen gebildet ist.

Hierdurch kann ein einfaches Bedienelement eingesetzt werden, das unmittelbar logisch verwendbar ist und gleichzeitig eine stabile Verbindung zwischen dem Bedienelement und der Schlüsselfläche bereitstellt.

Die Aufgaben der Erfindung werden auch gelöst durch ein Ventilsystem umfassen einen solchen Drehventilkörper und einen Ventilsitz, in dem der Drehventilkörper drehbar gelagert ist, wobei die Dichtnocke oder die Dichtnocken des Drehventilkörpers durch den Ventilsitz gestaucht sind.

Dabei kann vorgesehen sein, dass der Ventilsitz aus Kunststoff gefertigt ist, vorzugsweise aus thermoplastischem Kunststoff, besonders bevorzugt aus Polyamid, Polyetherketon und/oder Polyimid.

Durch diesen Aufbau kann das gesamte Ventil bestehend aus Ventilsitz und Drehventilkörper aus kostengünstigen Materialien gefertigt werden.

Ferner kann vorgesehen sein, dass im Ventilsitz zumindest zwei Öffnungen, vorzugsweise zumindest drei Öffnungen angeordnet sind, die in Gasleitungen münden, wobei eine Dichtnocke in einer ersten Position des Drehventilkörpers im Ventilsitz zumindest eine Öffnung verschließt und in einer zweiten gedrehten Position des Drehventilkörpers im Ventilsitz die Durchführung zumindest zwei Öffnungen verbindet, so dass eine durchgehende Gasleitung gebildet wird.

Dieser Aufbau stellt eine einwandfreie Bedienbarkeit und Handhabbarkeit des fertig aufgebauten Drehventils sicher.

Gemäß einer bevorzugten Weiterbildung des Ventils kann vorgesehen sein, dass im Ventilsitz zumindest drei Öffnungen die in Gasleitungen münden, wobei die erste Dichtnocke in einer ersten Position des Drehventilkörpers im Ventilsitz zumindest eine Öffnung verschließt und in einer zweiten gedrehten Position des Drehventilkörpers im Ventilsitz die Durchführung zumindest zwei Öffnungen verbindet, so dass eine durchgehende Gasleitung gebildet wird, und in der ersten Position die dritte Öffnung des Drehventilkörpers mit einer der Öffnungen im Ventilsitz verbunden ist und in der zweiten gedrehten Position des Drehventilkörpers im Ventilsitz die zweite Dichtnocke diese Öffnung im Ventilsitz verschließt

Auch diese Maßnahmen dienen der Verbesserung der Funktionalität des aufgebauten Drehventils.

Die der Erfindung zugrundeliegenden Aufgaben werden schließlich auch gelöst durch eine medizinische Austragsvorrichtung zum einmaligen Gebrauch, insbesondere Druckgasgetriebene medizinische Knochenzement-Austragsvorrichtung Sprühvorrichtung oder Vakuumerzeugungsvorrichtung, mit einem solchen Ventilsystem zur Regulierung des Gasstroms.

Der Erfindung liegt die überraschende Erkenntnis zugrunde, dass es durch die Verwendung elastischer Dichtnocken gelingt einen kostengünstigen Drehventilkörper aufzubauen mit dem eine Druck-dichte Abdichtung von Gasleitungen für komprimierte Gase möglich ist. Dazu dichten die flexiblen elastischen Dichtnocken die Öffnungen, in die die Gasleitungen einer Auspressvorrichtung münden, auch gegen Drücke ab, die bei der Verwendung von CO₂-Patronen auftreten können.

Um keine zu großen Drücke zu erzeugen, kann vorgesehen sein, dass ein Überdruckventil vorgesehen ist, dass sich ab einem vorherbestimmten Druck öffnet. Als vorherbestimmter Druck kann beispielsweise ein Druck von 50 bar verwendet werden.

Erfindungsgemäß ist auch ein Drehventil mit einem bereichsweise zylinderförmigen Ventilsitz, wobei der Drehventilkörper die folgenden Merkmale umfasst:
a) einen zylindrischen Grundkörper,
b) eine senkrecht zur Mantelfläche des Zylinders durch den Zylinder gehende gasdurchlässige Öffnung,
c) eine an der äußeren Mantelfläche angeordnete Dichtnocke, die sich über den Außendurchmesser des Zylinders erhebt, und neben der Öffnung im gleichen Abstand wie die Öffnung zur Stirnfläche des Zylinders angeordnet ist, wobei der Außendurchmesser der Dichtnocke größer ist als der Innendurchmesser des Ventilsitzes,
d) wenigstens eine Schlüsselfläche, die an den Stirnseiten des Zylinders angeordnet ist und die mit wenigstens einem Bedienelement verbindbar ist.

Erfindungsgemäß ist auch ein Drehventil mit zylinderförmigen Ventilsitz, wobei der Drehventilkörper aufgebaut ist aus
a) einem Zylinder, mit zumindest einem von einer Stirnfläche des Zylinders ausgehenden axialen Hohlraum, der mit der Umgebung gasdurchlässig verbunden ist,
b) wenigstens zwei an der äußeren Zylinderoberfläche tangential umlaufende Nuten,
c) wenigstens zwei Führungsringen, die in den Nuten angeordnet sind,
d) eine senkrecht zur Mantelfläche des Zylinders durch den Zylinder gehende gasdurchlässige erste Öffnung,
e) eine gasdurchlässige zweite Öffnung, welche die äußere Mantelfläche mit dem axialen Hohlraum gasdurchlässig verbindet,
e) eine an der äußeren Mantelfläche angeordnete erste Dichtnocke, die sich über den Außendurchmesser des Zylinders erhebt, und neben der ersten Öffnung im gleichen Abstand wie die erste Öffnung zur Stirnfläche des Zylinders angeordnet ist, wobei der Außendurchmesser der ersten Dichtnocke größer ist als der Innendurchmesser des Ventilsitzes,
f) eine an der äußeren Mantelfläche angeordneten zweite Dichtnocke, die sich über den Außendurchmesser des Zylinders erhebt, und neben der zweiten Öffnung im gleichen Abstand wie die zweite Öffnung zur Stirnfläche des Zylinders angeordnet ist, wobei der Außendurchmesser der zweiten Dichtnocke größer ist als der Innendurchmesser des Ventilsitzes,
g) wobei die Öffnungen in einem Mindestwinkel von 10 ° versetzt sind,
h) wobei die Dichtnocken in einem Mindestwinkel von 10 ° versetzt sind,
i) wobei die Öffnungen diagonal zueinander angeordnet sind,
j) wobei die Dichtnocken diagonal zueinander angeordnet sind,
k) wobei die Diagonale zwischen den Öffnungen die Diagonale der Dichtnocken so schneidet, dass die Abstände der Öffnungen zu dem Schnittpunkt gleich lang sind, wie der Abstand der Dichtnocken zum Schnittpunkt, und
l) dass an den Stirnseiten des Zylinders wenigstens eine Schlüsselfläche angeordnet ist, die mit wenigstens einem Bedienelement verbunden werden kann.

Erfindungsgemäß kann auch vorgesehen sein, dass die gesamte Oberfläche der Dichtnocken einen Ausschnitt einer Mantelfläche eines Zylinders bilden, der einen größeren Außendurchmesser besitzt als der Zylinder. Der Außendurchmesser der Dichtnocken ist kleiner als der Innendurchmesser des Ventilsitzes. Durch die kreisförmige Oberfläche der Dichtnocken ist die Dichtnockenoberfläche ausreichend glatt, um einerseits eine Dichtfunktion zu gewährleisten und andererseits ein Gleiten entlang des Ventilsitzes. Der größere Außendurchmesser der Dichtnocken gegenüber dem Zylinder führt dazu, dass die Nockenoberfläche gegen den Ventilsitz gepresst wird. Erst durch diese Anpressung ist es möglich, eine wirksame Abdichtung auch bei Gasdrücken von 60 bar zu erzielen, wie sie bei Verwendung von flüssigem Kohlendioxid auftreten.

Erfindungsgemäß ist ferner, dass an dem Ventilkörper eine Schlüsselfläche ausgebildet ist, die formschlüssig mit dem Bedienelement verbunden ist. Das bedeutet, dass bei Bewegung des Bedienelements, zum Beispiel einem Abzugsgriff, diese Bewegung durch den Formschluss mit der Schlüsselfläche auf das Ventilelement übertragen werden kann, so dass das Ventilelement im Ventilsitz gedreht wird und dadurch der Druckgasstrom an- und abgeschaltet werden kann.

Es ist für die vorliegende Erfindung von Bedeutung, dass bei der Schlüsselfläche, die Bewegung des Bedienelements direkt auf das Ventilelement ohne Schlupf übertragen wird. Deshalb soll die Schlüsselfläche hinsichtlich ihrer Form so ausgebildet sein, dass ein Schlupf ausgeschlossen ist.

Die Schlüsselfläche kann daher dreieckig, quadratisch, rechteckig, sechseckig, achteckig oder ellipsenförmig sein. Es ist auch möglich, Schlüsselflächen durch Kombinationen von Quadraten, Rechtecken, Dreiecken, Ellipsen oder Kreisen zu bilden.

Erfindungsgemäß ist ferner, dass der Ventilsitz und der Ventilkörper bevorzugt aus Kunststoff bestehen, wobei thermoplastischer Kunststoff ganz besonders bevorzugt ist. Dadurch kann das Ventil kostengünstig durch Spritzgießen hergestellt werden.

Der Ventilsitz besteht bevorzugt aus steifen Polymeren, die nur ein sehr geringes Kriechvermögen zeigen. Dabei sind Polyamid, Polyetherketon und Polyimid bevorzugt. Als besonders geeignet hat sich dazu Polyamid 12 erwiesen.

Das Drehventil besteht ebenfalls aus einem Kunststoff. Hierbei haben sich Polyoxymethylen, Polyamid und Teflon als geeignet erwiesen. Besonders geeignet ist Polyoxymethylen, weil dieses Polymer eine sehr hohe Formbeständigkeit besitzt und andererseits eine ausreichend hohe Rückstellkraft besitzt. Polyoxymethylen hat weiterhin den Vorteil, dass es nur vernachlässigbar kriecht. Polyoxymethylen zeigt weiterhin ein sehr gutes Reibungsverhalten. Daher ist Polyoxymethylen besonders bevorzugt zur Fertigung der Dichtnocken oder des gesamten Drehventils.

Das Drehventil ist zur Regulierung des Gasstroms in medizinischen Geräten, insbesondere in Austragsvorrichtungen für Knochenzement zum einmaligen Gebrauch bestimmt. Als medizinische Geräte kommen insbesondere Auspressvorrichtungen nach der DE 10 2010 019 222 A1 sowie der DE 10 2010 019 224 B3, Sprühvorrichtungen (wie der nicht vorveröffentlichten DE 10 2012 013 464) und Vorrichtungen zur temporären Vakuumerzeugung (wie der nicht vorveröffentlichten DE 10 2011 117 527) in Betracht.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: zwei schematische perspektivische Seitenansichten eines erfindungsgemäßen Drehventilkörpers von gegenüberliegenden Seiten;
- Figur 2:: eine Seitenansicht eines erfindungsgemäßen Drehventilkörpers;
- Figur 3:: eine Querschnittansicht entlang der Drehachse eines erfindungsgemäßen Drehventilkörpers;
- Figur 4:: eine Querschnittansicht entlang der Drehachse eines erfindungsgemäßen Drehventilkörpers in der Gegenrichtung zu Figur 3;
- Figur 5:: eine schematische Querschnittansicht durch eine Dichtnocke und der angrenzenden Bereiche eines erfindungsgemäßen Drehventilkörpers; und
- Figur 6:: eine schematische Querschnittansicht einer erfindungsgemäßen Austragsvorrichtung mit einem erfindungsgemäßen Drehventil.

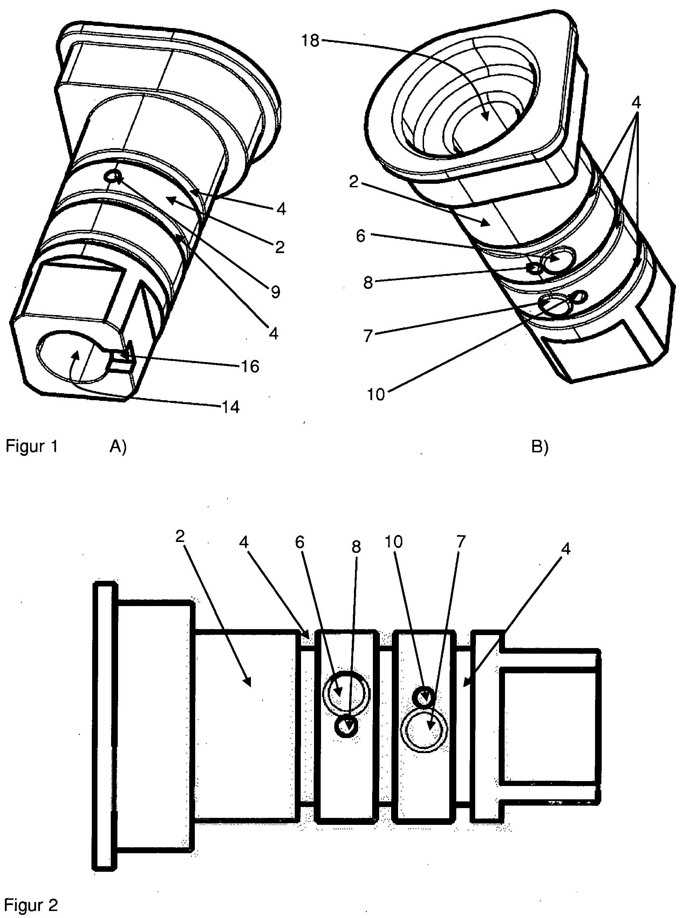

Figur 1 zeigt zwei schematische perspektivische Seitenansichten eines erfindungsgemäßen Drehventilkörpers von gegenüberliegenden Seiten. Der Drehventilkörper umfasst eine zylindrische Mantelfläche 2, die im eingebauten Zustand (nicht gezeigt) an einem Ventilsitz einer medizinischen Austragsvorrichtung anliegen soll. In der zylindrischen Mantelfläche 2 sind Nuten 4 angeordnet, die zur Aufnahme von Führungsringen vorgesehen sind, mit denen der Drehventilkörper im Ventilsitz während der Drehung positioniert wird. Die Führungsringe können auch fest mit dem Ventilsitz verbunden sein.

Die zylindrische Mantelfläche 2 kann auch leicht konisch ausgeführt werden. Auf den Mantelflächen 2 zwischen den Nuten 4 ist eine erste Dichtnocke 6 und eine zweite Dichtnocke 7 aus Polyoxymethylen angeordnet. Im eingebauten Zustand dienen diese dazu, die Öffnungen zweier Gasleitungen im Ventilsitz abhängig von der Drehung des Drehventilkörpers druckdicht zu verschließen.

Neben der ersten Dichtnocke 6 ist eine erste Öffnung 8 angeordnet, die in einen Durchgang mündet und sich bis auf die Rückseite des Drehventilkörpers (Figur 1 A) erstreckt und dort in eine zweite Öffnung 9 in der Mantelfläche 2 auf der gegenüberliegenden Seite des Drehventilkörpers mündet. Neben der zweiten Dichtnocke 7 ist eine dritte Öffnung 10 angeordnet. Diese verbindet einen Durchgang mit einer vierten Öffnung (in den Figuren 1 A) und 1B) nicht zu sehen), der in einem Hohlraum 14 des Drehventilkörpers angeordnet ist. In einer der Wandungen des Hohlraums 14 ist eine Ausnehmung 16 angeordnet.

Die Drehachse des Drehventils ist die Zylinderachse der zylindrischen Mantelflächen 2. Bezogen auf diese Drehachse sind die erste Dichtnocke 6 und die erste Öffnung 8 in der gleichen Höhe angeordnet. In gleicher Weise ist die zweite Dichtnocke 7 und die dritte Öffnung 10 in der gleichen Höhe bezogen auf die Drehachse des Drehventils angeordnet. Die erste Dichtnocke 6 und die erste Öffnung 8 sind genau wie die zweite Dichtnocke 7 und die Dritte Öffnung 10 um 10° versetzt bezogen auf die Drehachse des Drehventils zueinander angeordnet.

Auf der dem Hohlraum 14 gegenüberliegenden Stirnfläche des Drehventilkörpers ist ein zweiter Hohlraum 18 angeordnet. Die beiden Enden im Bereich der Stirnflächen des Drehventilkörpers weisen eine gebrochene Drehsymmetrie auf, und können so als Schlüsselelemente für Bedienelemente dienen.

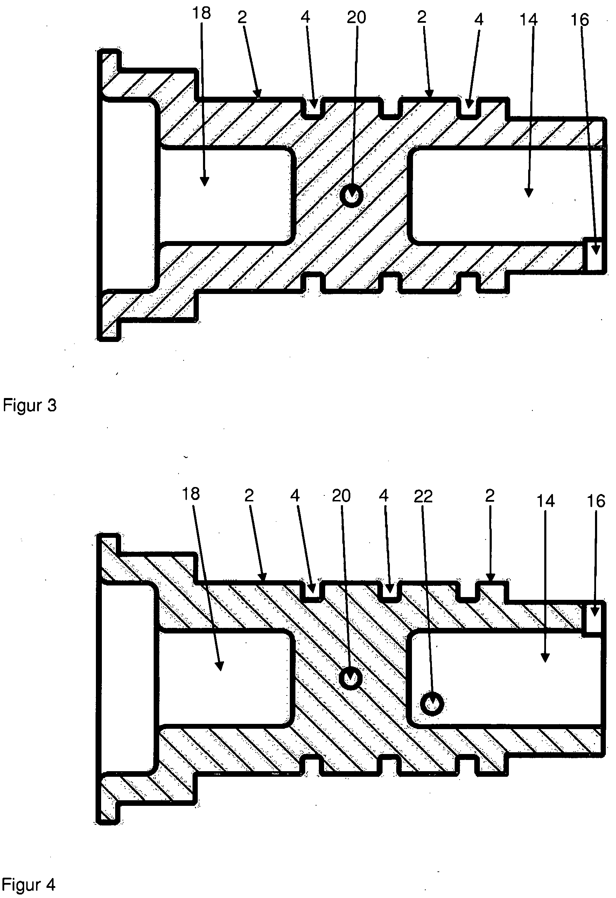

Figur 2 zeigt eine Seitenansicht des erfindungsgemäßen Drehventilkörpers nach Figur 1. In den Figuren 3 und 4 sind Querschnittansichten entlang der Drehachse des Drehventilkörpers dargestellt. Darin ist im Inneren des Drehventilkörpers die Durchführung 20 zu erkennen, die sich durch den Drehventilkörper erstreckt und die erste Öffnung 8 mit der zweiten Öffnung 9 verbindet. Des Weiteren ist in Figur 4. Des Weiteren ist in Figur 4 die vierte Öffnung 22 in dem ersten Hohlraum 14 zu erkennen.

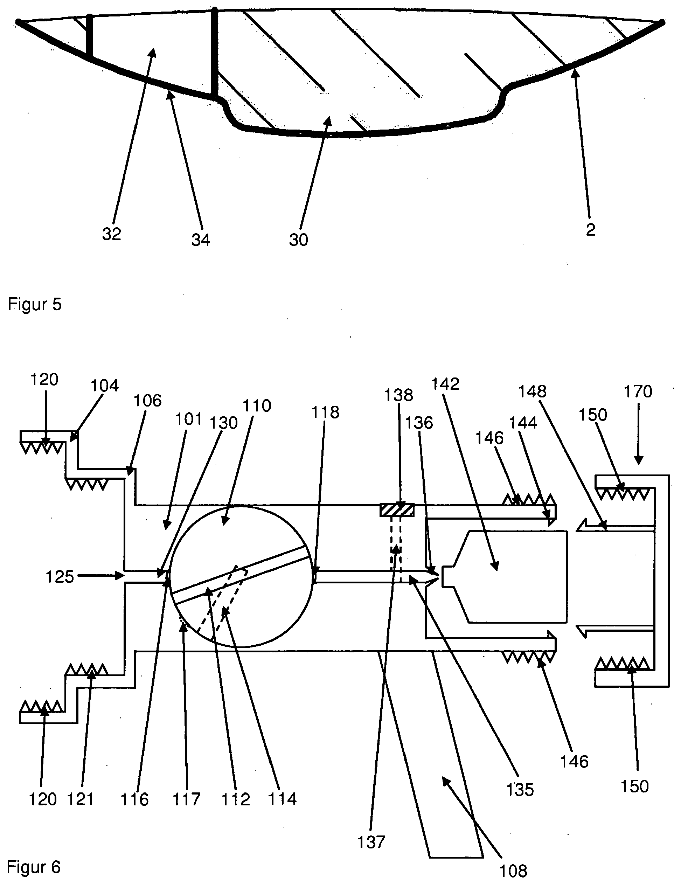

Figur 5 zeigt eine schematische Querschnittansicht durch eine Dichtnocke 30 und die angrenzenden Bereiche eines erfindungsgemäßen Drehventilkörpers in einer geschnittenen Ansicht. Neben der Dichtnocke 30 ist eine Öffnung 34 angeordnet, die in eine Durchführung 34 mündet. Die Dichtnocke 30 ist mit dem Drehventilkörper einteilig aus einem flexiblen Kunststoff ausgebildet. Die Dichtnocke 30 erhebt sich nur geringfügig über den Durchmesser des drehsymmetrischen Mantels des Drehventilkörpers, auf dem die Dichtnocke 30 angeordnet ist. Im Ventilsitz wird diese Dichtnocke vom Ventilsitz zusammengedrückt und dient dazu eine Öffnung im Ventilsitz druckdicht zu verschließen.

Figur 6 zeigt eine Querschnittansicht einer erfindungsgemäßen Austragsvorrichtung mit einem Grundkörper 101 an dessen Vorderseite ein erstes Kartuschenanschlussstück 104 mit großem Durchmesser für Kartuschen mit großem Durchmesser und ein zweites Kartuschenanschlussstück 106 mit kleinerem Durchmesser für Kartuschen mit kleinerem Durchmesser angeordnet sind. An den Zylinderinnenwänden der Kartuschenanschlussstücke 104, 106 sind Fixierungsvorrichtungen 120, 121 in Form von Innengewinden zur Fixierung von Kartuschen mit Außengewinden (nicht gezeigt) vorgesehen. Die Fixierungsvorrichtungen 120, 121 können auch durch Gewinde, Gewindeausschnitte oder Zapfen zum Arretieren der Kartuschen in den Kartuschenanschlussstücken 104, 106 realisiert sein.

In der rückseitigen Wand des zweiten, inneren Kartuschenanschlussstücks 106 befinden sich drei Öffnungen 125, die zu drei Durchgängen im Grundkörper 101 führen. In den Kartuschenanschlussstücken 104, 106 fixierte Kartuschen schließen dicht mit den Kartuschenanschlussstücken 104, 106 ab. Dadurch wird gewährleistet, dass ein Gasdruck, der durch eine der Öffnungen 125 auf den Boden einer fixierten Kartuschen gerichtet wird, auch auf diesen Kartuschenboden wirken kann, und nicht zwischen den Kartuschen und den Kartuschenanschlussstücken 104, 106 entweicht. Hierzu können Dichtungselemente (nicht gezeigt) in den Kartuschenanschlussstücken 104, 106 und/oder an den Kartuschen, insbesondere an den Kartuschenböden, vorgesehen sein. Es kann aber auch ausreichend sein, eine dichte Fixierungsvorrichtung 120, 121 vorzusehen, die beispielsweise durch ein Gewinde realisiert sein kann.

In einer Öffnung im Grundkörper 101 befindet sich ein Drehventilkörper 110 mit rotationssymmetrischer Geometrie, der um seine Symmetrieachse (senkrecht zur in Figur 6 dargestellten Schnittebene) drehbar im Grundkörper 101 gelagert ist. In dem Ventilkörper befindet sich ein erster Durchgang 112, der sich gerade durch den Drehventilkörper 110 erstreckt und ein zweiter Durchgang 114, der abgewinkelt ist und in einer Stirnfläche des Drehventilkörpers 110 durch einen Sterilfilter (nicht gezeigt) nach außen mündet. Um 20° versetzt zu den Öffnungen der Durchgänge 112, 114 im Zylindermantel des Drehventilkörpers 110 befinden sich Dichtnocken 116, 117, 118.

Im vorderen Teil des Grundkörpers 101 (zu den Kartuschenanschlussstücken 104, 106 gerichtet) ist eine vordere Gasleitung 130 angeordnet, die in eine Öffnung 125 zu den Kartuschenanschlussstücken 104, 106 mündet.

Im hinteren Teil des Grundkörpers 101 sind zwei hintere Gasleitungen 135 angeordnet, die in Öffnungen zum Drehventilkörper 110 münden. Mit den Dichtnocken 116, 117, 118 sind die Öffnungen zu den Gasleitungen 130, 135 im Ventilsitz des Drehventils 110 gasdicht und bis zu einem Druck von 70 bar Druckdicht verschließbar. Auf der der Rückseite der Austragsvorrichtung zugewandten Seite des Grundkörpers 101 mündet eine der zwei hinteren Gasleitungen 135 in einen hohlen, beidseitig geöffneten Dorn 136 mit einem Durchlass. Der zweite hintere Kanal 135 mündet in einen Ablasskanal 137, der über einen Sterilfilter 138 mit der Umgebung der Austragsvorrichtung verbunden ist. Mit diesem Ablasskanal kann ein im Bereich der Kartuschenanschlussstücke auf dem Förderkolben einer angeschlossenen Kartusche (nicht gezeigt) lastende Gasdruck abgelassen werden. Dazu ist im Drehventilkörper 110 ein weiterer Durchgang (nicht gezeigt) angeordnet, über den bei geeigneter Stellung des Drehventilkörpers eine der Öffnungen 125 mit der Gasleitung 137 verbunden werden kann. Zum Verschließen der Öffnungen im Ventilsitz hierzu sind zusätzliche Dichtnocken (nicht gezeigt) vorgesehen.

Im Drehventilkörper 110 sind dann drei entlang der Symmetrieachse des Drehventilkörpers 110 versetzte Durchgänge 112, 114, die zusätzlich um diese Symmetrieachse gegeneinander verdreht sind, angeordnet. Die Durchgänge 112, 114 sind derart im Drehventilkörper 110 angeordnet und die Gasleitungen 130, 135, 137 derart im Grundkörper 101 angeordnet, dass bei drei Stellungen des Drehventilkörpers 110, die durch eine Drehung des Drehventilkörpers 110 gegen den Grundkörper 101 im Ventilsitz einstellbar sind, die vordere Gasleitung 130 mit jeweils einer hinteren Gasleitung 135, 137 über einen der Durchgänge 112, 114 oder dem dritten Durchgang (nicht gezeigt) miteinander verbunden sind, wobei alle anderen Gasleitungen 130, 135, 137 durch die Dichtnocken 116, 117, 118 des Drehventilkörpers 110 durchdicht geschlossen sind. Die Breite der vorderen Öffnung zum Drehventilkörper 110, die in die vordere Gasleitung 130 mündet, reicht dazu aus, dass jeder der Durchgänge 112, 114 mit der vorderen Gasleitung 130 verbindbar ist.

Alternativ können auch drei vordere Gasleitungen 130 im vorderen Teil des Grundkörpers 101 vorgesehen sein, wobei jede vordere Gasleitung 130 über jeweils einen Durchgang 112, 114 mit einer hinteren Gasleitung 135, 137 verbindbar ist. Jeder der drei vorderen Gasleitungen 130 ist dann eine hintere Gasleitung 135, 137 und ein Durchgang 112, 114 zugeordnet. Vor dem Sterilfilter 138 und/oder dem Sterilfilter im Drehventilkörper 110 kann ein Überdruckventil angeordnet sein, dass sich ab einem gewissen Druck öffnet.

Am Drehventilkörper 110 ist auf einer Seite, die aus dem Grundkörper 101 herausragt, ein Schlüsselelement (nicht gezeigt) angeordnet, in den ein Bedienelement (nicht gezeigt) mit passendem Gegenstück zum Drehen des Drehventils 110 im Ventilsitz des Grundkörpers 101 greift.

Der Dorn 136 ragt in einen Hohlraum im hinteren Teil des Grundkörpers 101, der zur Aufnahme einer Druck-Gaspatrone 142 geeignet ist. Der Dorn 136 dient dazu, eine Gaspatrone 142, deren Öffnungsseite auf den Dorn 136 gedrückt wird, zu öffnen. Am hinteren Ende des Hohlraums ist innenseitig eine Arretierungsvorrichtung 144 und außenseitig am Grundkörper 101 ein Befestigungsmittel 146 in Form eines Außengewindes angeordnet. Die Arretierungsvorrichtung 144 dient dazu, eine ungewollte Bewegung der Verschlusskappe 170 zu verhindern. Die Arretierungsvorrichtung 144 kann dazu in Arretierungshaken 148 greifen, die an der Verschlusskappe 170 angeordnet sind. Das Befestigungsmittel 146 dient dazu, die Verschlusskappe 170 mit dem Grundkörper 101 zu verbinden, dabei die Gaspatrone 142 auf den Dorn 136 zu drücken und so zu öffnen. Dazu ist an der Verschlusskappe 170 ein Befestigungsmittel 150 in Form eines Innengewindes vorgesehen.

### Bezugszeichenliste

- 2: Mantelfläche
- 4: Nut
- 6, 7, 30: Dichtnocke
- 8, 9, 10, 22, 34: Öffnung
- 14, 18: Hohlraum
- 16: Aussparung
- 20, 32, 112, 114: Durchführung
- 101: Grundkörper
- 104: Kartuschenanschlussstück mit großem Durchmesser
- 106: Kartuschenanschlussstück mit kleinem Durchmesser
- 108: Pistolengriff
- 110: Drehventilkörper
- 116, 117, 118: Dichtnocke
- 120, 121: Fixierungsvorrichtung
- 125: Öffnung im Kartuschenanschlussstück
- 130, 135: Gasleitung
- 136: Hohler Dorn
- 137: Ablasskanal
- 138: Sterilfilter
- 142: Gaspatrone
- 144: Arretierungsvorrichtung
- 146: Befestigungsmittel
- 148: Arretierungshaken
- 150: Befestigungsmittel
- 170: Verschlusskappe

## Patentansprüche

1. Drehventilkörper (110) für eine druckgasbetriebene Austragsvorrichtung für Knochenzemente der drehbar in einem passenden Ventilsitz in der Austragsvorrichtung anzuordnen ist, wobei der Drehventilkörper (110) in zumindest einem für den Ventilsitz vorgesehenen Bereich um die Drehachse des Drehventilkörpers (110) drehsymmetrisch ist und wobei in dem Drehventilkörper (110) zumindest eine Durchführung (20, 32, 112) angeordnet ist, **dadurch gekennzeichnet, dass** auf der drehsymmetrischen Mantelfläche (2) dieses Bereichs zumindest eine Dichtnocke (6, 30, 116) aus einem elastischen Kunststoff angeordnet ist, die sich über den Radius der drehsymmetrischen Mantelfläche (2) erhebt, so dass der Umfang des Drehventilkörpers (110) durch die Dichtnocke (6, 30, 116) größer als der Innendurchmesser des Ventilsitzes ist, und dass sich die zumindest eine Durchführung von einer ersten Öffnung (8, 34) in der drehsymmetrischen Mantelfläche (2) des Drehventilkörpers (110) im Bereich der Dichtnocke (6, 30, 116) bis in eine zweite Öffnung (9) in der drehsymmetrischen Mantelfläche (2) des Drehventilkörpers (110) erstreckt.

2. Drehventilkörper (110) nach Anspruch 1, **dadurch gekennzeichnet, dass** auf der drehsymmetrischen Mantelfläche (2) zumindest eine zweite Dichtnocke (7, 117) aus einem elastischen Kunststoff angeordnet ist, die sich über den Radius der drehsymmetrischen Mantelfläche (2) erhebt, so dass der Umfang des Drehventilkörpers (110) durch die zweite Dichtnocke (7, 117) größer als der Innendurchmesser des Ventilsitzes ist, und wobei in dem Drehventilkörper (110) zumindest eine zweite Durchführungen (114) angeordnet ist, wobei sich die zweite Durchführung (114) von einer dritten Öffnung (10) in der drehsymmetrischen Mantelfläche (2) des Drehventilkörpers (110) im Bereich der zweiten Dichtnocke (7, 117) bis in eine vierte Öffnung (22) in der in Bezug auf die Austragsvorrichtung nach außen gerichteten Deckfläche des Drehventilkörpers (110) erstreckt.

3. Drehventilkörper (110) nach Anspruch 2, **dadurch gekennzeichnet, dass** in zumindest einer der Deckflächen des Drehventilkörpers (110) ein axialer Hohlräum (14, 18) angeordnet ist, der nach außen offen ist, wobei die vierte Öffnung (22) in dem Hohlraum (14) angeordnet ist, wobei vorzugsweise der Hohlraum (14) in der in Bezug auf die Austragsvorrichtung nach außen gerichteten Deckfläche des Drehventilkörpers (110) angeordnet ist.

4. Drehventilkörper (110) nach einem der Ansprüche 1 bis 3, **dadurch gekennzeichnet, dass**
der gesamte Drehventilkörper (110) zu mehr als 90%, vorzugsweise vollständig aus Kunststoff besteht, vorzugsweise aus dem gleichen Kunststoff aus dem auch die Dichtnocke (6, 30, 116) oder die Dichtnocken (6, 7, 30, 116, 117, 118) bestehen, wobei der Drehventilkörper (110) und die Dichtnocke (6, 30, 116) oder die Dichtnocken (6, 7, 30, 116, 117, 118) besonders bevorzugt einteilig aufgebaut sind.

5. Drehventilkörper (110) nach einem der vorangehenden Ansprüche, **dadurch gekennzeichnet, dass**
die Dichtnocke (6, 30, 116) oder die Dichtnocken (6, 7, 30, 116, 117, 118) aus Polyoxymethylen, Polyamid oder Teflon besteht oder bestehen und vorzugsweise auch der Drehventilkörper (110) zu mehr als 90%, insbesondere vollständig aus Polyoxymethylen, Polyamid oder Teflon besteht.

6. Drehventilkörper (110) nach einem der vorangehenden Ansprüche, **dadurch gekennzeichnet, dass**
die erste Öffnung (8, 34), bezogen auf die Drehachse des Drehventilkörpers (110), in der gleichen Höhe wie die erste Dichtnocke (6, 30, 116) angeordnet ist und vorzugsweise die dritte Öffnung (10), bezogen auf die Drehachse des Drehventilkörpers (110), in der gleichen Höhe wie die zweite Dichtnocke (7, 117) angeordnet ist.

7. Drehventilkörper (110) nach einem der vorangehenden Ansprüche, **dadurch gekennzeichnet, dass**
sich die erste Durchführung (20, 32, 112) senkrecht zur Drehachse des Drehventilkörpers (110) durch den Drehventilkörper (110) erstreckt, so dass die erste Öffnung (8, 34) gegenüberliegend zur zweiten Öffnung (9) angeordnet ist.

8. Drehventilkörper (110) nach einem der vorangehenden Ansprüche, **dadurch gekennzeichnet, dass**
in der drehsymmetrischen Mantelfläche (2) des Drehventilkörpers (110) zumindest zwei tangential umlaufende Nuten (4) vorgesehen sind, wobei vorzugsweise in den Nuten (4) Führungsringe, besonders bevorzugt Kunststoffführungsringe angeordnet sind.

9. Drehventilkörper (110) nach einem der Ansprüche 2 bis 8, **dadurch gekennzeichnet, dass**
die erste Öffnung (8, 34) und die dritte Öffnung (10) bezogen auf die Drehachse des Drehventilkörpers (110) um mindestens 10° zueinander versetzt und in unterschiedlicher Höhe angeordnet sind, insbesondere zwischen 10° und 90° zueinander versetzt angeordnet sind.

10. Drehventilkörper (110) nach einem der Ansprüche 2 bis 9, **dadurch gekennzeichnet, dass**
die erste Dichtnocke (6, 30, 116) und die zweite Dichtnocke (7, 117) bezogen auf die Drehachse des Drehventilkörpers (110) um mindestens 10° zueinander versetzt und in unterschiedlicher Höhe angeordnet sind, insbesondere zwischen 15° und 90° zueinander versetzt angeordnet sind, wobei vorzugsweise die erste Dichtnocke (6, 30, 116) bezogen auf die Drehachse in der gleichen Höhe wie die erste Öffnung (8, 34) angeordnet ist, die zweite Dichtnocke (7, 117) bezogen auf die Drehachse in der gleichen Höhe wie die dritte Öffnung (10) angeordnet ist und die Dichtnocken (6, 7, 30, 116, 117) zu den in der gleichen Höhe angeordneten Öffnungen (8, 9, 10, 34) um den gleichen Winkel zueinander versetzt angeordnet sind, wie die Dichtnocken (6, 7, 30, 116, 117) zueinander.

11. Drehventilkörper (110) nach einem der Ansprüche 2 bis 9, **dadurch gekennzeichnet, dass**
in der in Bezug auf die Austragsvorrichtung nach außen gerichteten Deckfläche des Drehventilkörpers (110) wenigstens ein zur Drehachse des Drehventilkörpers (110) nicht symmetrisches Schlüsselelement angeordnet ist, so dass der Drehventilkörper (110) in dem Ventilsitz mit einem Bedienelement, das formschlüssig in das Schlüsselelement greift, drehbar ist.

12. Ventilsystem umfassend einen Drehventilkörper (110) nach einem der vorangehenden Ansprüche und einen Ventilsitz, in dem der Drehventilkörper (110) drehbar gelagert ist, wobei die Dichtnocke (6, 30, 116) oder die Dichtnocken (6, 7, 30, 116, 117, 118) des Drehventilkörpers (110) durch den Ventilsitz gestaucht sind.

13. Ventilsystem nach Anspruch 12, **dadurch gekennzeichnet, dass** der Ventilsitz aus Kunststoff gefertigt ist, vorzugsweise aus thermoplastischem Kunststoff, besonders bevorzugt aus Polyamid, Polyetherketon und/oder Polyimid.

14. Ventilsystem nach Anspruch 12 oder 13, **dadurch gekennzeichnet, dass** im Ventilsitz zumindest zwei Öffnungen, vorzugsweise zumindest drei Öffnungen angeordnet sind, die in Gasleitungen (130, 135) münden, wobei eine Dichtnocke (6, 30, 116) in einer ersten Position des Drehventilkörpers (110) im Ventilsitz zumindest eine Öffnung verschließt und in einer zweiten gedrehten Position des Drehventilkörpers (110) im Ventilsitz die Durchführung (20, 112) zumindest zwei Öffnungen verbindet, so dass eine durchgehende Gasleitung gebildet wird.

15. Ventilsystem nach Anspruch 12 oder 13, **dadurch gekennzeichnet, dass** im Ventilsitz zumindest drei Öffnungen die in Gasleitungen (130, 135, 137) münden, wobei die erste Dichtnocke (6, 30, 116) in einer ersten Position des Drehventilkörpers (110) im Ventilsitz zumindest eine Öffnung verschließt und in einer zweiten gedrehten Position des Drehventilkörpers (110) im Ventilsitz die Durchführung (20, 112) zumindest zwei Öffnungen verbindet, so dass eine durchgehende Gasleitung gebildet wird, und in der ersten Position die dritte Öffnung (10) des Drehventilkörpers (110) mit einer der Öffnungen im Ventilsitz verbunden ist und in der zweiten gedrehten Position des Drehventilkörpers (110) im Ventilsitz die zweite Dichtnocke (7, 117) diese Öffnung im Ventilsitz verschließt

16. Medizinische Austragsvorrichtung zum einmaligen Gebrauch, insbesondere Druckgasgetriebene medizinische Knochenzement-Austragsvorrichtung Sprühvorrichtung oder Vakuumerzeugungsvorrichtung, mit einem Ventilsystem nach einem der Ansprüche 12 bis 15 zur Regulierung des Gasstroms.

## Claims

1. Rotary valve body (110) for a compressed gas-operated dispensing device for bone cements that is to be arranged rotatably in a fitting valve seat in the dispensing device, whereby the rotary valve body (110) is rotationally symmetrical about the rotation axis of the rotary belt body (110), at least in a region provided for the valve seat, **characterised in that** at least one sealing cam (6, 30, 116) made of an elastic plastic material is arranged on the rotationally symmetrical jacket surface (2) of said region and is elevated above the radius of the rotationally symmetrical jacket surface (2) such that the circumference of the rotary valve body (110) is larger than the internal diameter of the valve seat due to the sealing cam (6, 30, 116), and whereby at least one feed-through (20, 32, 112) is arranged in the rotary valve body (110) and **in that** the at least one feed-through extends from a first opening (8, 34) in the rotationally symmetrical jacket surface (2) of the rotary valve body (110) in the region of the sealing cam (6, 30, 116) into a second opening (9) in the rotationally symmetrical jacket surface (2) of the rotary valve body (110).

2. Rotary valve body (110) according to claim 1, **characterised in that** at least one second sealing cam (7, 117) made of an elastic plastic material is arranged on the rotationally symmetrical jacket surface (2) and is elevated above the radius of the rotationally symmetrical jacket surface (2) such that the circumference of the rotary valve body (110) is larger than the internal diameter of the valve seat due to the second sealing cam (7, 117), and whereby at least one second feed-through (114) is arranged in the rotary valve body (110), whereby the second feed-through (114) extends from a third opening (10) in the rotationally symmetrical jacket surface (2) of the rotary valve body (110) in the region of the second sealing cam (7, 117) into a fourth opening (22) in the cover surface of the rotary valve body (110) that faces outward with respect to the dispensing device.

3. Rotary valve body (110) according to claim 2, **characterised in that** an axial hollow space (14, 18) that is open to the outside is arranged in at least one of the cover surfaces of the rotary valve body (110), whereby the fourth opening (22) is arranged in the hollow space (14), whereby the hollow space (14) preferably is arranged in the cover surface of the rotary valve body (110) that faces outward with respect to the dispensing device.

4. Rotary valve body (110) according to any one of the claims 1 to 3,
**characterised in that**
more than 90%, preferably all, of the entire rotary valve body (110) consist of plastic material, preferably of the same plastic material of which the sealing cam (6, 30, 116) or the sealing cams (6, 7, 30, 116, 117, 118) consist, whereby the rotary valve body (110) and the sealing cam (6, 30, 116) or the sealing cams (6, 7, 30, 116, 117, 118) particularly preferably are designed as a single part.

5. Rotary valve body (110) according to any one of the preceding claims,
**characterised in that**
the sealing cam (6, 30, 116) or the sealing cams (6, 7, 30, 116, 117, 118) consists or consist of polyoxymethylene, polyamide or Teflon and, preferably more than 90%, in particular all, of the rotary valve body (110) consist of polyoxymethylene, polyamide or Teflon as well.

6. Rotary valve body (110) according to any one of the preceding claims,
**characterised in that**
the first opening (8, 34) is arranged, with respect to the rotation axis of the rotary valve body (110), at the same height as the first sealing cam (6, 30, 116) and, preferably, the third opening (10) is arranged, with respect to the rotation axis of the rotary valve body (110), at the same height as the second sealing cam (7, 117).

7. Rotary valve body (110) according to any one of the preceding claims,
**characterised in that**
the first feed-through (20, 32, 112) extends perpendicular to the rotation axis of the rotary valve body (110) through the rotary valve body (110) such that the first opening (8, 34) is arranged opposite to the second opening (9).

8. Rotary valve body (110) according to any one of the preceding claims,
**characterised in that**
at least two tangentially-circumferential grooves (4) are provided in the rotationally symmetrical jacket surface (2) of the rotary valve body (110) whereby, preferably, guide rings, particularly preferably plastic guide rings, are arranged in the grooves (4).

9. Rotary valve body (110) according to any one of the claims 2 to 8,
**characterised in that**
the first opening (8, 34) and the third opening (10) are arranged at an offset from each other by at least 10° with respect to the rotation axis of the rotary valve body (110) and are arranged at different heights, in particular are arranged at an offset between 10° and 90° with respect to each other.

10. Rotary valve body (110) according to any one of the claims 2 to 9,
**characterised in that**
the first sealing cam (6, 30, 116) and the second sealing cam (7, 117) are arranged at an offset from each other by at least 10° with respect to the rotation axis of the rotary valve body (110) and at different heights, in particular are arranged at an offset between 15° and 90° with respect to each other, whereby the first sealing cam (6, 30, 116) preferably is arranged at the same height as the first opening (8, 34) with respect to the rotation axis, the second sealing cam (7, 117) is arranged at the same height as the third opening (10) with respect to the rotation axis, and the sealing cams (6, 7, 30, 116, 117) are arranged at an offset from each other with respect to the openings (8, 9, 10, 34), which are arranged at the same height, by the same angle at which the sealing cams (6, 7, 30, 116, 117) are arranged at an offset with respect to each other.

11. Rotary valve body (110) according to any one of the claims 2 to 9, **characterised in that**
at least one key element that is not symmetrical to the rotation axis of the rotary valve body (110) is arranged in the cover surface of the rotary valve body (110) that faces outward with respect to the dispensing device, such that the rotary valve body (110) can be rotated in the valve seat by means of an operating element that engages the key element in a form-fitting manner.

12. Valve system comprising a rotary valve body (110) according to any one of the preceding claims and a valve seat, in which the rotary valve body (110) is supported like in a bearing such as to be rotatable, whereby the sealing cam (6, 30, 116) or the sealing cams (6, 7, 30, 116, 117, 118) of the rotary valve body (110) are compressed by the valve seat.

13. Valve system according to claim 12, **characterised in that** the valve seat is made of plastic material, preferably of thermoplastic material, particularly preferably of polyamide, polyetherketone and/or polyimide.

14. Valve system according to claim 12 or 13, **characterised in that** at least two openings, preferably at least three openings, are arranged in the valve seat and merge into gas lines (130, 135), whereby a sealing cam (6, 30, 116), in a first position of the rotary valve body (110) in the valve seat, closes at least one opening and, in a second rotated position of the rotary valve body (110) in the valve seat, the feed-through (20, 112) connects at least two openings such that a through-going gas line is formed.

15. Valve system according to claim 12 or 13, **characterised in that** at least three openings that merge into gas lines (130, 135, 137) [are arranged] [A1] in the valve seat, whereby the first sealing cam (6, 30, 116), in a first position of the rotary valve body (110) in the valve seat, closes at least one opening and, in a second rotated position of the rotary valve body (110) in the valve seat, the feed-through (20, 112) connects at least two openings such that a through-going gas line is formed, and, in the first position, the third opening (10) of the rotary valve body (110) is connected to one of the openings in the valve seat and, in the second rotated position of the rotary valve body (110) in the valve seat, the second sealing cam (7, 117) closes said opening in the valve seat.

16. Medical dispensing device for single use, in particular compressed gas-driven medical bone cement dispensing device, spray device or vacuum generating device having a valve system according to any one of the claims 2 to 15 for regulation of the gas flow.

## Revendications

1. Corps de soupape rotative (110) pour un dispositif de distribution exploité par gaz comprimé pour des ciments osseux qui est à disposer à rotation dans un siège de soupape adapté dans le dispositif de distribution, dans lequel le corps de soupape rotative (110) est à symétrie de rotation dans au moins une région prévue pour le siège de soupape autour de l'axe de rotation du corps de soupape rotative (110) et dans lequel au moins un passage (20, 32, 112) est disposé dans le corps de soupape rotative (110), **caractérisé en ce qu'**au moins une came d'étanchéité (6, 30, 116) constituée d'un plastique élastique est disposée sur la face d'enveloppe à symétrie de rotation (2) de cette région, laquelle s'élève au-delà du rayon de la face d'enveloppe à symétrie de rotation (2) de sorte que la périphérie du corps de soupape rotative (110) à travers la came d'étanchéité (6, 30, 116) est supérieure au diamètre interne du siège de soupape, et que l'au moins un passage s'étend d'une première ouverture (8, 34) dans la face d'enveloppe à symétrie de rotation (2) du corps de soupape rotative (110) dans la région de la came d'étanchéité (6, 30, 116) jusque dans une deuxième ouverture (9) dans la face d'enveloppe à symétrie de rotation (2) du corps de soupape rotative (110).

2. Corps de soupape rotative (110) selon la revendication 1, **caractérisé en ce qu'**au moins une seconde came d'étanchéité (7, 117) constituée d'un plastique élastique est disposée sur la face d'enveloppe à symétrie de rotation (2), laquelle s'élève au-delà du rayon de la face d'enveloppe à symétrie de rotation (2) de sorte que la périphérie du corps de soupape rotative (110) à travers la seconde came d'étanchéité (7, 117) est supérieure au diamètre interne du siège de soupape, et dans lequel au moins un second passage (114) est disposé dans le corps de soupape rotative (110), dans lequel le second passage (114) s'étend d'une troisième ouverture (10) dans la face d'enveloppe à symétrie de rotation (2) du corps de soupape rotative (110) dans la région de la seconde came d'étanchéité (7, 117) jusque dans une quatrième ouverture (22) dans la face de recouvrement orientée vers l'extérieur par rapport au dispositif de distribution du corps de soupape rotative (110).

3. Corps de soupape rotative (110) selon la revendication 2, **caractérisé en ce que**
un espace creux axial (14, 18) est disposé dans au moins une des faces de recouvrement du corps de soupape rotative (110), lequel est ouvert vers l'extérieur, dans lequel la quatrième ouverture (22) est disposée dans l'espace creux (14), dans lequel l'espace creux (14) est de préférence disposé dans la face de recouvrement orientée vers l'extérieur par rapport au dispositif de distribution du corps de soupape rotative (110).

4. Corps de soupape rotative (110) selon une des revendications 1 à 3, **caractérisé en ce que**
le corps de soupape rotative entier (110) se compose à plus de 90 %, de préférence entièrement, de plastique, de préférence du même plastique duquel la came d'étanchéité (6, 30, 116) ou les cames d'étanchéité (6, 7, 30, 116, 117, 118) se composent également, dans lequel le corps de soupape rotative (110) et la came d'étanchéité (6, 30, 116) ou les cames d'étanchéité (6, 7, 30, 116, 117, 118) sont construits de manière particulièrement préférée d'une pièce.

5. Corps de soupape rotative (110) selon une des revendications précédentes, **caractérisé en ce que**
la came d'étanchéité (6, 30, 116) ou les cames d'étanchéité (6, 7, 30, 116, 117, 118) se compose ou se composent de polyoxyméthylène, polyamide ou téflon et le corps de soupape rotative (110) se compose de préférence également à plus de 90 %, notamment entièrement, de polyoxyméthylène, polyamide ou téflon.

6. Corps de soupape rotative (110) selon une des revendications précédentes, **caractérisé en ce que**
la première ouverture (8, 34) est disposée à la même hauteur que la première came d'étanchéité (6, 30, 116) par rapport à l'axe de rotation du corps de soupape rotative (110), et la troisième ouverture (10) est de préférence disposée à la même hauteur que la seconde came d'étanchéité (7, 117) par rapport à l'axe de rotation du corps de soupape rotative (110).

7. Corps de soupape rotative (110) selon une des revendications précédentes, **caractérisé en ce que**
le premier passage (20, 32, 112) s'étend perpendiculairement à l'axe de rotation du corps de soupape rotative (110) à travers le corps de soupape rotative (110) de sorte que la première ouverture (8, 34) est disposée à l'opposé de la deuxième ouverture (9).

8. Corps de soupape rotative (110) selon une des revendications précédentes, **caractérisé en ce que**
au moins deux rainures périphériques de manière tangentielle (4) sont prévues dans la face d'enveloppe à symétrie de rotation (2) du corps de soupape rotative (110), dans lequel des anneaux de guidage, de manière particulièrement préférée des anneaux de guidage en plastique, sont de préférence disposés dans les rainures (4).

9. Corps de soupape rotative (110) selon une des revendications 2 à 8, **caractérisé en ce que**
la première ouverture (8, 34) et la troisième ouverture (10) sont disposées de manière décalée d'au moins 10° l'une par rapport à l'autre et à hauteur différente par rapport à l'axe de rotation du corps de soupape rotative (110), sont notamment disposées de manière décalée entre 10° et 90° l'une par rapport à l'autre.

10. Corps de soupape rotative (110) selon une des revendications 2 à 9, **caractérisé en ce que**
la première came d'étanchéité (6, 30, 116) et la seconde came d'étanchéité (7, 117) sont disposées de manière décalée d'au moins 10° l'une par rapport à l'autre et à hauteur différente par rapport à l'axe de rotation du corps de soupape rotative (110), sont notamment disposées de manière décalée entre 15° et 90° l'une par rapport à l'autre, dans lequel la première came d'étanchéité (6, 30, 116) est de préférence disposée à la même hauteur que la première ouverture (8, 34) par rapport à l'axe de rotation, la seconde came d'étanchéité (7, 117) est disposée à la même hauteur que la troisième ouverture (10) par rapport à l'axe de rotation, et les cames d'étanchéité (6, 7, 30, 116, 117) sont disposées de manière décalée du même angle les unes par rapport aux autres que les cames d'étanchéité (6, 7, 30, 116, 117) les unes par rapport aux autres, par rapport aux ouvertures (8, 9, 10, 34) disposées à la même hauteur.

11. Corps de soupape rotative (110) selon une des revendications 2 à 9, **caractérisé en ce que**
au moins un élément de clé non symétrique à l'axe de rotation du corps de soupape rotative (110) est disposé dans la face de recouvrement orientée vers l'extérieur par rapport au dispositif de distribution du corps de soupape rotative (110) de sorte que le corps de soupape rotative (110) est rotatif dans le siège de soupape avec un élément de commande qui s'engrène par conjugaison de formes dans l'élément de clé.

12. Système de soupape comprenant un corps de soupape rotative (110) selon une des revendications précédentes et un siège de soupape, dans lequel le corps de soupape rotative (110) est logé à rotation, dans lequel la came d'étanchéité (6, 30, 116) ou les cames d'étanchéité (6, 7, 30, 116, 117, 118) du corps de soupape rotative (110) sont comprimées à travers le siège de soupape.

13. Système de soupape selon la revendication 12, **caractérisé en ce que** le siège de soupape est fabriqué en plastique, de préférence en plastique thermoplastique, de manière particulièrement préférée en polyamide, polyéthercétone et/ou polyimide.

14. Système de soupape selon la revendication 12 ou 13, **caractérisé en ce que** au moins deux ouvertures, de préférence au moins trois ouvertures, sont disposées dans le siège de soupape, lesquelles débouchent dans des conduites de gaz (130, 135), dans lequel une came d'étanchéité (6, 30, 116) ferme au moins une ouverture dans une première position du corps de soupape rotative (110) dans le siège de soupape et le passage (20, 112) connecte au moins deux ouvertures dans une seconde position tournée du corps de soupape rotative (110) dans le siège de soupape de sorte qu'une conduite de gaz continue est formée.

15. Système de soupape selon la revendication 12 ou 13, **caractérisé en ce que** au moins trois ouvertures dans le siège de soupape, lesquelles débouchent dans des conduites de gaz (130, 135, 137), dans lequel la première came d'étanchéité (6, 30, 116) ferme au moins une ouverture dans une première position du corps de soupape rotative (110) dans le siège de soupape et le passage (20, 112) connecte au moins deux ouvertures dans une seconde position tournée du corps de soupape rotative (110) dans le siège de soupape de sorte qu'une conduite de gaz continue est formée, et la troisième ouverture (10) du corps de soupape rotative (110) est reliée à une des ouvertures dans le siège de soupape dans la première position et la seconde came d'étanchéité (7, 117) ferme cette ouverture dans le siège de soupape dans la seconde position tournée du corps de soupape rotative (110).

16. Dispositif de distribution médical à usage unique, notamment dispositif de distribution de ciment osseux médical exploité par gaz comprimé, dispositif de pulvérisation ou dispositif de génération de vide, avec un système de soupape selon une des revendications 12 à 15 pour la régulation du courant gazeux.
